# FASCICULE DE BREVET EUROPEEN

(11) **EP 0 998 580 B1**
(45) Date de publication et mention de la délivrance du brevet: **05.04.2006**
(21) Numéro de dépôt: 98938731.1
(22) Date de dépôt: 10.07.1998
(51) Int. Cl.: C12Q 1/04, C12Q 1/44

(54) **MILIEU DE CULTURE PERMETTANT LA DETECTION DES BACTERIES PATHOGENES DU GENRE LISTERIA ET PROCEDE D'IDENTIFICATION DE CES BACTERIES**
KULTURMEDIUM FÜR DEN NACHWEIS VON BAKTERIELLER PATHOGENE DER GATTUNG LISTERIA, SOWIE VERFAHREN ZUR IDENTIFIZIERUNG VON DIESER BAKTERIEN
CULTURE MEDIUM FOR DETECTING PATHOGENIC BACTERIA OF THE GENUS LISTERIA AND METHOD FOR IDENTIFYING SAID BACTERIA

(30) Priorité: 15.07.1997 FR 9708960
(43) Date de publication de la demande: 10.05.2000
(73) Titulaire: Bio-Rad Pasteur, 92430 Marnes-la-Coquette (FR)
(72) Inventeur: FACON, Jean-Pierre, F-62840 Fleurbaix (FR); SIMON, Frédéric, F-59114 Terdeghem (FR)
(74) Mandataire: Bernasconi, Jean Raymond
(86) Numéro de dépôt international: PCT/FR1998/001516
(87) Numéro de publication internationale: WO 1999/004032

(56) Documents cités:
- EP-A- 0 326 062
- EP-A- 0 496 680
- GOLDFINE H ET AL: "PURIFICATION AND CHARACTERIZATION OF LISTERIA -MONOCYTOGENES PHOSPHATIDYLINOSITOL -SPECIFIC PHOSPHOLIPASE C." INFECT IMMUN 60 (10). 1992. 4059-4067. CODEN: INFIBR ISSN: 0019-9567, XP002059616
- MENGAUD J ET AL: "IDENTIFICATION OF PHOSPHATIDYLINOSITOL -SPECIFIC PHOSPHOLIPASE C ACTIVITY IN LISTERIA -MONOCYTOGENES A NOVEL TYPE OF VIRULENCE FACTOR." MOL MICROBIOL 5 (2). 1991. 367-372. CODEN: MOMIEE ISSN: 0950-382X, XP002059617
- NOTERMANS S H W ET AL: "PHOSPHATIDYLINOSITOL -SPECIFIC PHOSPHOLIPASE C ACTIVITY AS A MARKER TO DISTINGUISH BETWEEN PATHOGENIC AND NONPATHOGENIC LISTERIA -SPP." APPL ENVIRON MICROBIOL 57 (9). 1991. 2666-2670. CODEN: AEMIDF ISSN: 0099-2240, XP002059618

## Description

La présente invention concerne un milieu de culture permettant le dénombrement et l'identification directe des bactéries pathogènes du genre *Listeria* ainsi qu'un procédé mettant en oeuvre ce milieu.

L'isolement et l'identification de la bactérie *Listeria monocytogenes* est un problème majeur de la surveillance de l'hygiène agro-alimentaire et de la bactériologie médicale. Parmi les bactéries du genre *Listeria spp.* seule l'espèce *monocytogenes* est connue pour être pathogène pour l'homme. Les autres espèces *de Listeria* ne sont pas pathogènes ou sont pathogènes seulement pour les animaux. C'est le cas notamment de *Listeria ivanovii.* On sait par ailleurs qu'il est possible d'inhiber la croissance des *Listeria monocytogenes* en utilisant des bactériocines. Par exemple, la demande de brevet EP 326 062 décrit un procédé d'inhibition des *Listeria monocytogenes* qui met en oeuvre une bactériocine provenant des *Pediococcus acidilactici.*

La voie de contamination par *Listeria monocytogenes* est le plus souvent d'origine alimentaire. Ainsi, il est nécessaire de prévoir la détection de *Listeria monocytogenes* tout au long de la filière agro-alimentaire, des matières premières en passant par l'environnement des ateliers de fabrication jusqu'au produit fini destiné à la consommation.

Dans le cadre de diagnostic d'affections bactériennes chez l'humain, il est également important de distinguer la *Listeria monocytogenes* parmi les autres bactéries du genre *Listeria spp.* qui ne sont pas pathogènes.

La détection et l'isolement de *Listeria spp.* sont classiquement réalisés avec des milieux de culture sélectifs. Les milieux sélectifs Oxford (Curtis et al., *Lett. Appl. Microbiol.* (1989), 8, pp. 85-98) et Palcam (Van Netten et al., *J. Food Microbiol.* (1988), 6, pp. 187-188) sont les plus couramment utilisés. Ces milieux permettent la détection de toutes les espèces du genre *Listeria spp..* Ainsi, les colonies typiques observées doivent être soumises à des tests d'identification supplémentaires, tels que des tests microscopiques, et/ou biochimiques, et/ou immunologiques, et/ou génétiques, de manière à vérifier l'appartenance à l'espèce *monocytogenes.* Or, les manipulations supplémentaires, nécessaires à l'identification de *Listeria monocytogenes,* augmentent la durée et le coût des analyses. Elles nécessitent une multitude de réactifs et l'intervention de personnel qualifié. De plus, le prélèvement des colonies soumises à l'identification étant aléatoire, les manipulations supplémentaires sont souvent source d'erreur ou au moins, la cause d'une moindre précision et fiabilité. C'est le cas notamment, lorsque sur le milieu d'isolement, les colonies de *Listeria monocytogenes* sont très minoritaires par rapport aux colonies formées par les autres espèces de *Listeria*

La demande de brevet EP 496 680 décrit un procédé d'analyse bactériologique pour différencier la *Listeria monocytogenes* des autres bactéries du genre *Listeria spp.*

Selon ce procédé, on utilise un milieu d'identification comprenant un substrat chromogène ou fluorigène susceptible d'être hydrolysé par la glycine aminopeptidase. Le milieu utilisé peut également contenir éventuellement un substrat de fermentation et/ou un substrat réductible et/ou un substrat hydrolysable par voie enzymatique (tel que le substrat de l'α-mannosidase) dont la transformation chimique permet de caractériser l'espèce *Listeria* présente dans l'échantillon à analyser.

On sait par ailleurs qu'il est possible de discriminer les espèces pathogènes de *Listeria, Listeria monocytogenes* et *Listeria ivanovii,* des autres *Listeria* par la mise en évidence de l'activité phosphatidylinositol spécifique phospholipase C spécifique du phosphatidylinositol (PIPLC).

En effet, il a été démontré que la PIPLC est sécrétée dans le milieu de culture des espèces pathogènes du genre *Listeria* telles que *Listeria monocytogenes* et *Listeria ivanovii* (Leimeister-Wächter et al, *Mol. Microbiol.* (1991) 5(2), pp. 361-366 ; J. Mengaud et al, *Mol. Microbiol.* (1991) 5(2), pp. 367-372 et Goldfine et al, *Infection and Immunity* (1992) 60(10), pp. 4059-4067). On sait également qu'il est possible d'identifier ces deux espèces pathogènes grâce à des procédés indirects (Notermans et al, *App. and Env. Microbiology* (1991), vol 57 n° 9, pp. 2666-2670). Selon le procédé proposé par Notermans et al, la souche de *Listeria* à tester, préalablement isolée, est ensemencée sous forme de tache à la surface d'une gélose TY (Tryptone Yeast Extract). Après 24 à 48 heures d'incubation à 37°C, une deuxième couche de gélose est coulée dans les boîtes de Pétri. Cette deuxième couche contient de l'agarose, du chloramphénicol et du L-α-phosphatidyl-inositol, substrat naturel de la PIPLC. Les boîtes sont ensuite de nouveau incubées à 37°C et observées pendant 5 jours. Ce procédé nécessite donc plusieurs étapes, telles que l'isolement de la souche, l'ensemencement en tache sur gélose et la répartition d'une seconde couche de gélose contenant le substrat de la PIPLC. En outre, ce procédé ne permet pas de distinguer les bactéries *Listeria monocytogenes,* pathogènes pour l'homme, des bactéries *Listeria ivanovii* qui, comme indiqué précédemment, ne sont pas pathogènes pour l'homme.

L'objet de l'invention est un milieu permettant l'identification des bactéries pathogènes du genre *Listeria* en une seule étape.

La présente invention concerne plus spécialement un milieu de culture spécifique, permettant le dénombrement et l'identification directe des espèces pathogènes du genre *Listeria,* ledit milieu contenant un substrat chromogène de synthèse spécifiquement clivé par la PIPLC, notamment le 5-bromo-4-chloro-3-indolyl-phosphatidyl-myoinositol et contenant en outre du sang ou un de ses dérivés, de préférence du sérum. On utilise ce substrat chromogène de préférence sous forme de sel, par exemple sous forme de sel de sodium, de potassium ou d'ammonium. Parmi les sels préférés est l'ammonium de 5-bromo-4-chloro-3-indolyl-myoinositol-1-yl-phosphate.

Le milieu, selon l'invention, permet la distinction directe entre, d'une part, *Listeria monocytogenes* et *Listeria ivanovii* qui forment des colonies colorées et d'autre part, les autres espèces de *Listeria* dont les colonies sont non colorées.

Selon un mode préféré de réalisation de l'invention, l'utilisation du 5-bromo-4-chloro-3-indolyl-phosphatidyl-myoinositol permet la détection des espèces *Listeria monocytogenes* et *Listeria ivanovii* qui forment des colonies bleues, les autres espèces de *Listeria* restant non colorées.

De manière préférentielle, le milieu de culture nutritif gélosé, selon l'invention, contient le 5-bromo-4-chloro-3-indolyl-phosphatidyl-myoinositol sous forme libre ou sous forme de sel, à une concentration de 100 à 500 mg/l, de préférence à une concentration de 150 à 300 mg/l.

Le milieu de culture nutritif gélosé utilisé dans l'invention doit permettre la croissance des *Listeria spp..* Il est par exemple possible d'utiliser le gélose Columbia, constituée de peptones, d'amidon, de chlorure de sodium et d'agar et bien connue de l'homme du métier.

La présente invention concerne aussi l'utilisation du 5-bromo-4-chloro-3-indolyl-phosphatidyl-myoinositol ou un de ses sels pour la préparation d'un milieu de culture spécifique, permettant le dénombrement et l'identification directe des espèces pathogènes du genre *Listeria* et notamment de la bactérie *Listeria monocytogenes.*

De manière étonnante il a été aussi trouvé, que l'addition du sang ou des ses dérivés, - tels que par exemple plasma ou sérum dans le milieu de culture permet d'obtenir des colonies PIPLC positives ayant une coloration très nette

La présente invention concerne également un milieu de culture nutritif gélosé spécifique, permettant le dénombrement et l'identification directe des espèces pathogènes du genre *Listeria* et notamment de la bactérie *Listeria monocytogenes,* ledit milieu contenant le 5-bromo-4-chloro-3-indolyl-phosphatidyl-myolnositol ou un de ses sels et du sang ou un de ses dérivés, de préférence du sérum.

La proportion du sang ou de ses constituants dans le milieu selon l'invention, peut être comprise entre 20 et 80 ml, plus spécialement entre 40 et 60 ml, de préférence 50 ml pour un litre de milieu de culture. On préfère des milieux de culture contenant du 5-bromo-4-chloro-3-indolyl-phosphatidylmyoinositol ou un de ses sels à une concentration de 100 à 200 mg/ml et du sérum à une proportion de 40 à 60 ml pour 1 litre de milieu.

L'addition dans le milieu de culture d'un agent pulvérulent tel que le kaolin ou la silice contribue à intensifier la coloration de culture de colonies de PIPLC positives, en rendant ainsi le test plus facile à interpréter.

De préférence l'agent pulvérulent est ajouté au milieu de culture complété avec du sérum.

Des milieux de culture nutritifs gélosé selon l'invention, contenant le 5-bromo-4-chloro-3-indolyl-phosphatidyl-myoinositol ou un de ses sels en tant que substrat chromogène de synthèse spécifiquement clive par la PIPLC, du sérum et un agent pulvérulent, tel que le kaolin ou la silice, font aussi partie de l'invention.

La concentration de l'agent pulvérulent dans le milieu de culture nutritif gélosé dépend de la nature de l'agent utilisé et peut varier entre 5 et 30 g/l, de préférence entre 15 et 25 g/l.

Selon l'invention, le milieu de culture peut également contenir un glucide (carbohydrate) métabolisable par *Listeria ivanovï* mais pas par *Listeria monocytogenes,* tel que le xylose par exemple. L'addition d'un tel glucide dans un milieu de culture contenant le 5-bromo-4-chloro-3-indolyl-phosphatidylmyoinositol ou un de ses sels permet alors la distinction entre d'une part, *Listeria monocytogenes* qui forme des colonies bleu franc, et d'autre part *Listeria ivanovii* qui forme des colonies bleu pâle à vertes et enfin les autres espèces de *Listeria* dont les colonies sont incolores. Des carbohydrates métabolisés par la *Listeria monocytogenes* sont décrits dans Manual of Clinical Microbiology (6^{th} Edition - 1995) ASM Press Washington D.C. pp. 343-344.

La présente invention concerne également un milieu de culture nutritif gélosé spécifique permettant le dénombrement et l'identification directe de *Listeria monocytogenes,* ledit milieu contenant le 5-bromo-4-chloro-3-indolyl-phosphatidyl-myoinositol ou un de ses sels en tant que substrat chromogène de synthèse spécifiquement clivé par la PIPLC, des dérivés du sang, de préférence le sérum, un agent pulvérulent tel que le kaolin ou la silice et un glucide métabolisable ou fermentescible par *Listeria ivanovii,* mais pas par *Listeria monocytogenes,* de préférence le xylose.

La concentration du glucide métabolisable par *Listeria ivanovii* mais pas par *Listeria monocytogenes,* dans le milieu de culture, peut varier entre 5 et 15 g/l et dépend bien entendu du glucide utilisé.

Le milieu de culture, selon l'invention, peut également contenir un indicateur de pH. Comme indicateurs du pH, on peut citer ceux qui sont couramment utilisés en microbiologie par exemple l'acide alizarinesulfonique, le rouge de méthyle, le rouge de chlorophénol, le tournesol, le pourpre de bromocrésol, le rouge de bromophénol, le bleu de bromoxylénol, l'alizarine, le bleu de bromothymol, le rouge de phénol, etc. Bien entendu, l'indicateur du pH doit être utilisé dans le milieu de culture en concentration adaptée. Cette dernière dépend de la nature de l'indicateur et peut varier entre 50 et 300 mg/l. Parmi les différents indicateurs de pH, on préfère utiliser le rouge de phénol. Cet indicateur améliore encore la distinction entre *Listeria monocytogenes* et *Listeria ivanovii,* les colonies étant, lorsque le milieu contient le 5-bromo-4-chloro-3-indolyl-phosphatidyl-myoinositol ou un de ses sels respectivement de couleur bleu franc sans virage de l'indicateur pour *Listeria monocytogenes* et de couleur bleu pâle et entourées d'un cercle jaune pour *Listeria ivanovii,* ce phénomène étant lié au virage de l'indicateur.

Selon l'invention, un milieu de culture préféré est un milieu de culture nutritif gélosé, contenant le 5-bromo-4-chloro-3-indolyl-phosphatidyl-myoinositol ou un de ses sels, des dérivés du sang, de préférence le sérum, un agent pulvérulent tel que le kaolin ou la silice, un glucide métabolisable par *Listeria ivanovii* mais pas par *Listeria monocytogenes,* de préférence le xylose et un indicateur de pH tel que le rouge de phénol.

D'autre part, l'activité PIPLC n'est pas présente exclusivement chez *Listeria monocytogenes* et *Listeria ivanovii.* Cette activité enzymatique est notamment rencontrée chez certaines espèces de *Bacillus* (Griffith et al., *Methods Enzymol.* (1991), 197, pp. 493-502) et de *Clostridium* (Taguchi et al., *Arch. Biochem. Biophys.* (1978), 186, pp 196-201). De plus, le développement d'une flore saprophyte importante (bactéries ou levures) sur le milieu de culture peut nuire à la détection des *Listeria monocytogenes* par un phénomène de compétition. Ainsi, il est souhaitable d'inhiber la croissance de germes interférents afin d'éliminer les risques de faux positifs (colonies bleues formées par des bactéries autres que *Listeria monocytogenes)* ou de faux négatifs (masquage de la coloration bleue des colonies de *Listeria monocytogenes* par un développement important d'autres contaminants). Pour éviter ces phénomènes, il est recommandé de compléter le milieu de culture avec des agents antibactériens et/ou antifongiques vis à vis desquels les *Listeria spp.* sont peu ou non sensibles. Ces agents peuvent être utilisés seuls ou en combinaison. Parmi ces agents, on peut citer par exemple le chlorure de lithium, le chlorhydrate d'acriflavine, l'acide nalidixique, la polymixine B, le cefotan, le sulfate de colistine, la fosfomycine, la ceftazimidine, le moxalactam, la cycloheximide, l'amphotéricine B.

Les milieux de culture nutritifs gélosés contenant du 5-bromo-4-chloro-3-indolyl-phosphatidyl-myoinositol ou un de ses sels, des dérivés du sang, de préférence le sérum, un agent pulvérulent tel que le kaolin ou la silice, un glucide métabolisable par *Listeria ivanovii* mais pas par Listeria monocytogenes, de préférence du xylose, un indicateur de pH tel que notamment le rouge de phénol et des agents antibactériens ou antifongiques font également partie de la présente invention.

La présente invention concerne aussi un procédé d'identification des bactéries pathogènes du genre *Listeria* comprenant :
- l'ensemencement d'un échantillon susceptible de contenir lesdites bactéries pathogènes du genre *Listeria* sur un milieu de culture gélosé contenant un substrat chromogène spécifique de la PIPLC,
- l'incubation dudit milieu de culture ensemencé avec ledit échantillon,
- et la détermination de la présence desdites bactéries pathogènes du genre *Listeria* par la couleur caractéristique du substrat. Ledit échantillon susceptible de contenir des bactéries pathogène du genre *Listeria* est ensemencé sous une forme brute ou est préalablement dilué ou est préalablement enrichi avant d'être ensemencé sur un milieu de culture, tel que défini précédemment.

Selon un mode préféré de réalisation du procédé selon l'invention, on cultive un échantillon brut à tester, dilué ou ayant subi une ou plusieurs phases d'enrichissement sur un milieu de culture nutritif gélosé qui contient un substrat chromogène de synthèse spécifiquement clivé par la PIPLC, qui est le 5-bromo-4-chloro-3-indolyl-phosphatidyl-myoinositol ou un de ses sels, des dérivés du sang tels que le sérum, un agent pulvérulent tel que le kaolin ou la silice, un glucide métabolisable par *Listeria ivanovii* mais pas par *Listeria monocytogenes,* de préférence le xylose et éventuellement un indicateur de pH tel notamment que le rouge de phénol et des agents antibactériens ou antifongiques. On détermine la présence de *Listeria monocytogenes* par la couleur caractéristique de ses colonies.

Les exemples suivants sont donnés pour illustrer l'invention.

### EXEMPLE 1 : Différenciation entre Listeria pathogènes, autres Listeria spp. et interférents.

### PREPARATION DES MILIEUX DE CULTURE

### Milieu 1 : milieu de base

- Gélose Columbia 39 g/l
- Chlorure de lithium 10 g/l
- Ceftazidime* 20 mg/l
- Moxalactam* 20 mg/l
- Polymixine B* 20 mg/l
- Polymixine B* 20 mg/l
*: composants thermolabiles

### Milieu 2 : milieu de base + 5-bromo-4-chloro-4-chloro-3-indolyl-phosphatidyl-myoinositol

La composition en gramme par litre de ce milieu est identique à celle du milieu 1, mais le milieu 2 contient en plus 300 mg/l de 5-bromo-4-chloro-3-indolyl-phosphatidyl-myoinositol, sous forme de sel d'ammonium (B-7404 5-bromo-4-chloro-3-indoxyl-myoinositol-1-yl-phosphate ammonium salt Biosynth Suisse).

La préparation de chaque milieu s'effectue comme suit :
- Dissolution des composants non thermolabiles dans de l'eau osmosée.
- Chauffage des mélanges jusqu'à ébullition.
- Ajustement, si nécessaire, du pH à 7,3 =/- 0,2.
- Répartition dans des flacons à raison de 95 ml/flacon.
- Autoclavage des flacons à 120°C pendant 15 minutes.
- Addition des composants thermolabiles, en solution stérile après refroidissement de la gélose à 47°C environ
- Répartition des milieux autoclavés dans des boites de Pétri stériles (diamètre 90 mm) à raison de 15 à 20 ml/boîte.

### SOUCHES TESTEES

Les souches testées sont les suivantes :
*Listeria spp. :*
   *Listeria monocytogenes*
   *Listeria ivanovii*
   *Listeria innocua*
   *Listeria seeligeri*
   *Listeria welshimeri*
Interférents :
   *Bacillus cereus*
   *Staphyloccocus aureus*
   *Candida tropicalis*

### MISE EN CULTURE - INCUBATION - LECTURE

Les souches sont repiquées dans des bouillons Trypto-Caséine-Soja puis incubées à 37°C.

Après 18 heures d'incubation, les bouillons sont dilués dans du diluant "Tryptone Sel",constitué de la peptone Tryptone à 1/100 et de chlorure de sodium à 8,5/1000 ; de manière à obtenir des suspensions comprises entre 10⁶ et 10⁷ cellules/ml.

Pour chaque suspension, on ensemence par isolement avec une öse stérile de 1 µl, une boîte de milieu 1 et une boîte de milieu 2. Les boites sont incubées à 37°C. Après 24 heures et 48 heures d'incubation, les boîtes sont lues. La lecture consiste en une observation de la couleur des colonies formées par chacune des souches sur chacun des milieux. Les résultats obtenus sont indiqués dans le tableau I.

**TABLEAU I**

| | **COULEUR DES COLONIES** | | | | | | **Distinction** |
|---|---|---|---|---|---|---|---|
| **MILIEUX** | ***Listeria* path.** | | ***Listeria* non pathogènes** | | | **Interférents** | ***Listeria* path. et non path.** |
| | ***L. monoc.*** | ***L. ivan.*** | ***L. inn.*** | ***L. seel.*** | ***L. welsh.*** | | |
| 1 | blanche | blanche | blanche | blanche | blanche | inhibition | impossible |
| 2 | bleue | bleue | blanche | blanche | blanche | inhibition | possible |

Le milieu 2 permet de différencier les espèces *Listeria,* pathogènes et non pathogènes. Par ailleurs, le supplément sélectif permet d'inhiber les bactéries *Bacillus cereus* et *Staphylococcus aureus,* potentiellement faux positifs sur le milieu de culture.

### EXEMPLE 2 : Différenciation entre Listeria espèces pathogènes et autres espèces Listeria

### PREPARATION DES MILIEUX DE CULTURE

### Milieu 2: milieu de base + 5-bromo-4-chloro-3-indolyl-phosphatidvl-mvoinositol

Le milieu 2 est tel que décrit dans l'exemple 1.

### Milieu 3 : milieu de base + 5-bromo-4-chloro-3-indolyl-phosphatidyl-myoinositol

La composition du milieu 3 est identique à celle du milieu 2 indiqué ci-dessus, mais ce milieu contient 150 mg/l de 5-bromo-4-chloro-3-indolyl-phosphatidyl-myoinositol sous forme de sel d'ammonium au lieu de 300 mg/l (milieu 2).

### Milieu 4 : milieu de base + 5-bromo-4-chloro-3-indolvi-phosphatidyl-myoinositol + sang

La composition du milieu 4 est identique à celle du milieu 3, mais ce milieu contient en plus 50 ml/l de sang de cheval. Pour la préparation du milieu, le sang est traité comme un composant thermolabile.

### Milieu 5 : milieu de base + 5-bromo-4-chloro-3-indolyl-phosphatidyl-myoinositol + sérum

La composition du milieu 5 est identique à celle du milieu 4, mais ce milieu contient 50 ml/l de sérum de cheval au lieu de sang.

### Milieu 6 : milieu de base + 5-bromo-4-chloro-3-indolyl-phosphatidyl-myoinositol + sérum + silice

La composition du milieu 6 est identique à celle du milieu 5, mais ce milieu contient en plus 20g/l de silice, composant non thermolabile.

La préparation de chaque milieu est effectuée comme décrit dans l'exemple 1.

### SOUCHES TESTEES

Les souches testées sont les suivantes :
*Listeria spp. :*
   *Listeria monocytogenes*
   *Listeria ivanovii*
   *Listeria innocua*
   *Listeria seeligeri*
   *Listeria welshimeri*

### MISE EN CULTURE - INCUBATION - LECTURE

Pour la mise en culture, l'incubation et la lecture il a été procédé comme décrit dans l'exemple 1. Les résultats obtenus sont indiqués dans le tableau II.

**TABLEAU II**

| | **COULEUR DES COLONIES** | | | | | **Distinction** |
|---|---|---|---|---|---|---|
| **MILIEUX** | ***Listeria* pathogènes** | | ***Listeria* non pathogènes** | | | ***Listeria* path. et non path.** |
| | ***L*. *monoc.*** | ***L*. *ivan.*** | ***L*. *inn*.** | **L. *seel*.** | ***L. welsh.*** | |
| 1 | bleue | bleue | blanche | blanche | blanche | bonne |
| 2 | bleutée | bleutée | blanche | blanche | blanche | moyenne |
| 3 | bleu franc | bleu franc | blanche | blanche | blanche | excellente |
| 4 | bleue | bleue | blanche | blanche | blanche | bonne |
| 5 | bleu franc | bleu franc | blanche | blanche | blanche | excellente |

Les résultats du tableau Il démontrent que pour effectuer une lecture sûre et précise du test, c'est-à-dire obtenir une bonne distinction entre les espèces *Listeria* pathogènes et les *Listeria* non pathogènes, il est souhaitable de compléter le milieu de culture par du sang, du sérum ou un mélange de sérum et de silice. L'addition au milieu du sang, du sérum ou d'un mélange de sérum et de silice contribue à intensifier fortement la coloration bleue des colonies PIPLC positives et permet ainsi de réduire de manière très significative la concentration en 5-bromo-4-chloro-3-indolyl-phosphatidyl-myoinositol dans le milieu de culture.

### EXEMPLE 3 : Différenciation entre Listeria monocytogenes et autres espèces Listeria y compris Listeria ivanovii.

### PREPARATION DES MILIEUX DE CULTURE

### Milieu 6 : milieu de base + 5-bromo-4-chloro-3-indolyl-phosphatidyl-myoinositol + sérum + silice

Le milieu 6 est tel que décrit dans l'exemple 2.

### Milieu 7 : milieu de base + 5-bromo-4-chloro-3-indolyl-phosphatidyl-myoinositol + sérum + silice + xylose

La composition du milieu 7 est identique à celle du milieu 6, décrit dans cet exemple, mais ce milieu contient en plus 10g/l de xylose, composant qui pour la préparation du milieu est considéré comme composant thermolabile.

### Milieu 8 : milieu de base + 5-bromo-4-chloro-3-indolyl-phosphatidyl-myoinositol + sérum + silice + xylose + rouge de phénol

La composition du milieu 8 est identique à celle du milieu 7 indiqué ci-dessus, mais ce milieu contient en plus 80 mg/l de rouge de phénol (composant non thermolabile).

La préparation de chaque milieu est effectuée comme décrit dans l'exemple 1.

### SOUCHES TESTEES

Les souches testées sont les suivantes :
*Listeria spp. :*
   *Listeria monocytogenes*
   *Listeria ivanovii*
   *Listeria innocua*
   *Listeria seeligeri*

### MISE EN CULTURE - INCUBATION - LECTURE

Pour la mise en culture, l'incubation et la lecture, il a été procédé comme décrit dans l'exemple 1. Les résultats obtenus sont indiqués dans le tableau III.

**TABLEAU III**

| | **COULEUR DES COLONIES** | | | | **Distinction** | **Distinction** |
|---|---|---|---|---|---|---|
| M**ILIEUX** | ***Listeria* pathogènes** | | ***Listeria* non path.** | | ***Listeria* path. et non path.** | ***Listeria monoc.* et autres *Listeria*** |
| | ***L. monoc.*** | ***L. ivan.*** | ***L. inn.*** | ***L. seel.*** | | |
| 1 | bleu franc | bleu franc | blanche | blanche | possible | impossible |
| 2 | bleu franc | bleu pâle à verte | blanche | blanche | possible | possible |
| 3 | bleu franc (-) | bleu pâle à verte (+) | blanche (-) | blanche (+) | possible | possible |

| | | | | | | |
|---|---|---|---|---|---|---|
| (-) : pas de virage de l'indicateur coloré, la gélose reste rouge autour des colonies (souche xylose -) | | | | | | |
| (+) : virage de l'indicateur coloré, le cercle jaune autour des colonies (souche xylose +) | | | | | | |

Les résultats du tableau III démontrent que l'addition dans le milieu de culture du xylose, de préférence en combinaison avec le rouge de phénol permet la distinction entre *Listeria monocytogenes* et toutes les autres espèces de *Listeria* en particulier *Listeria ivanovii.*

### EXEMPLE 4 : Mise en oeuvre du procédé selon l'invention

214 souches de genre *Listeria* fournies par le Centre de Référence *Listeria* (Institut Pasteur Paris) ont été testées sur le milieu 8.

Les souches correspondant aux espèces suivantes ont été utilisées :
12 souches de *Listeria innocua*
10 souches de *Listeria seeligeri*
10 souches de *Listeria welshimeri*
10 souches de *Listeria ivanovii*
1 souche de *Listeria grayi*
171 souches de *Listeria monocytogenes*

Pour la mise en culture, incubation et la lecture, il a été procédé comme décrit dans l'exemple 1.

Toutes les souches de *Listeria monocytogenes* ont été identifiées, ainsi que toutes les souches de *Listeria ivanovii,* et aucun résultat faux positif n'a été identifié.

Cette expérience met en évidence la spécificité et la fiabilité du procédé selon l'invention.

## Revendications

1. Milieu de culture nutritif permettant la croissance spécifique des bactéries du genre *Listeria,* et l'identification directe des bactéries pathogènes du genre Listeria,
ledit milieu contenant un substrat chromogène de synthèse spécifiquement clivé par la phospholipase C spécifique du phosphatidylinositol (PIPLC), et contenant en outre du sang ou un de ses dérivés, de préférence du sérum.

2. Milieu de culture selon la revendication 1, dans lequel la proportion du sang ou de ses dérivés est comprise entre 20 et 80 ml pour 1 litre de milieu de culture, plus spécialement entre 40 et 60 ml, de préférence 50 ml pour 1 litre de milieu de culture.

3. Milieu de culture selon l'une quelconque des revendications 1 ou 2 contenant en outre un agent pulvérulent tel que le kaolin ou la silice.

4. Milieu de culture selon la revendication 3, dans lequel la concentration de l'agent pulvérulent est comprise entre 5 et 30 g/l, de préférence entre 15 et 25 g/l.

5. Milieu de culture selon l'une des revendications 1 à 4, dans lequel ledit substrat chromogène est le 5-bromo-4-chloro-3-indolyl-phosphatidyl-myoinositol ou un de ses sels.

6. Milieu de culture selon la revendication 5, dans lequel le 5-bromo-4-chloro-3-indolyl-phosphatidyl-myoinositol ou un de ses sels est une concentration de 100 à 500 mg/l, de préférence à une concentration de 150 à 300 mg/l.

7. Milieu de culture selon l'une quelconque des revendications 1 à 6, contenant en outre un glucide métabolisable par *Listeria ivanovii* mais pas par *Listeria monocytogenes.*

8. Milieu de culture selon la revendication 7, dans lequel ledit glucide métabolisable par *Listeria ivanovii* mais par *Listeria monocytogenes* est le xylose.

9. Milieu de culture selon l'une des revendications 7 et 8, dans lequel la concentration dudit glucide est comprise entre 5 et 15 g/l.

10. Milieu de culture selon l'une des revendications 7 à 9, contenant en outre un indicateur de pH, de préférence le rouge de phénol.

11. Milieu de culture selon la revendication 10, dans lequel la concentration dudit indicateur de pH est comprise entre 50 et 300 mg/l.

12. Milieu de culture selon l'une quelconque des revendications 1 à 11, contenant un ou plusieurs agents antibactériens et/ou antifongiques, de préférence choisi parmi le chlorure de lithium, le chlorhydrate d'acriflavine, l'acide nalidixique, la polymixine B, le cefotan, le sulfate de colistine, la fosfomycine, la ceftazimidine, le moxalactam, la cycloheximide, l'amphotéricine B.

13. Procédé d'identification des bactéries pathogènes du genre *Listeria* comprenant :
- l'ensemencement d'un échantillon susceptible de contenir lesdites bactéries pathogènes du genre *Listeria* sur un milieu de culture gélosé selon l'une quelconque des revendications 1 à 12 ;
- l'incubation dudit milieu de culture ensemencé avec ledit échantillon:
- et la détermination de la présence desdites bactéries pathogènes du genre *Listeria* par la couleur caractéristique du substrat.

14. Procédé selon la revendication 13 dans lequel ledit échantillon susceptible de contenir des bactéries pathogènes est préalablement enrichi avant d'être ensemencé sur un milieu de culture selon l'une des revendications 1 à 12.

## Patentansprüche

1. Nährkulturmedium, das die spezifische Vermehrung von Bakterien der Gattung *Listeria* und die direkte Identifizierung pathogener Bakterien dieser Gattung erlaubt, ein chromogenes Synthesesubstrat, das von der für Phosphatidylinositol (PIPLC) spezifischen Phospholipase C spezifisch gespalten wird, und außerdem Blut oder eines von dessen Derivaten, vorzugsweise Serum, enthält.

2. Kulturmedium nach Anspruch 1, in welchem der Anteil an Blut oder dessen Derivaten 20 bis 80 ml, insbesondere zwischen 40 und 60 ml, und vorzugsweise 50 ml auf 1 Liter Kulturmedium beträgt.

3. Kulturmedium nach Anspruch 1 oder 2, das außerdem ein pulverförmiges Mittel wie Kaolin oder Siliciumdioxid enthält.

4. Kulturmedium nach Anspruch 3, in welchem die Konzentration des pulverförmigen Mittels 5 bis 30 g/l und vorzugsweise zwischen 15 und 25 g/l beträgt.

5. Kulturmedium nach einem Ansprüche 1 bis 4, in welchem das chromogene Substrat 5-Brom-4-chlor-3-indolyl-phosphatidylmyoinositol oder eines seiner Salze ist.

6. Kulturmedium nach Anspruch 5, in welchem die Konzentration von 5-Brom-4-chlor-3-indolyl-phosphatidylmyoinositol oder einem seiner Salze 100 bis 500 mg/l und vorzugsweise 150 bis 300 mg/l beträgt.

7. Kulturmedium nach einem der Ansprüche 1 bis 6, das außerdem ein Glucid enthält, das sich von *Listeria ivanovii,* aber nicht von *Listeria monocytogenes* verstoffwechseln lässt.

8. Kulturmedium nach Anspruch 7, in welchem das Glucid, das von *Listeria ivanovii,* aber nicht von *Listeria monocytogenes* verstoffwechselbar ist, Xylose ist.

9. Kulturmedium nach Anspruch 7 oder 8, in welchem die Glucidkonzentration 5 bis 15 g/l beträgt.

10. Kulturmedium nach einem der Ansprüche 7 bis 9, das darüber hinaus einen pH-Wert-Indikator, vorzugsweise Phenolrot, enthält.

11. Kulturmedium nach Anspruch 10, in welchem die Konzentration des pH-Wert-Indikators 50 bis 300 mg/l beträgt.

12. Kulturmedium nach einem der Ansprüche 1 bis 11, das ein oder mehrere Bakterizide und/oder Fungizide enthält, das/die vorzugsweise aus Lithiumchlorid, Acriflavinhydrochlorid, Nalidixinsäure, Polymixin B, Cefotan, Colistinsulfat, Fosfomycin, Ceftazimidin, Moxalactam, Cycloheximid und Amphotericin B ausgewählt ist/sind.

13. Verfahren zum Identifizieren pathogener Bakterien der Gattung *Listeria*, welches das
- Beimpfen mit einer Probe, die diese pathogenen Bakterien der Gattung *Listeria* enthalten kann, auf einem Gelosekulturmedium nach einem der Ansprüche 1 bis 12,
- Inkubieren des mit der Probe beimpften Kulturmediums und
- Bestimmen des Vorhandenseins der pathogenen Bakterien der Gattung *Listeria* durch die charakteristische Färbung des Substrats
umfasst .

14. Verfahren nach Anspruch 13, in welchem die Probe, die pathogene Bakterien enthalten kann, aufkonzentriert wird, bevor mit ihr ein Kulturmedium nach einem der Ansprüche 1 bis 12 beimpft wird.

## Claims

1. Nutritive culture medium allowing the specific growth of bacteria of the genus *Listeria* and the direct identification of pathogenic bacteria of the genus *Listeria,* the said medium containing a synthetic chromogenic substrate specifically cleaved by the specific phospholipase C of phosphatidylinositol (PIPLC), and additionally containing blood or one of its derivatives, preferably serum.

2. Culture medium according to Claim 1, in which the proportion of blood or of its derivatives is between 20 and 80 ml per litre of culture medium, more especially between 40 and 60 ml, preferably 50 ml per litre of culture medium.

3. Culture medium according to either of Claims 1 and 2, additionally containing a pulverulent agent such as kaolin or silica.

4. Culture medium according to Claim 3, in which the concentration of the pulverulent agent is between 5 and 30 g/l, preferably between 15 and 25 g/l.

5. Culture medium according to one of Claims 1 to 4, in which the said chromogenic substrate is 5-bromo-4-chloro-3-indolylphosphatidylmyoinositol or one of its salts.

6. Culture medium according to Claim 5, in which the 5-bromo-4-chloro-3-indolylphosphatidylmyoinositol or one of its salts is at a concentration of 100 to 500 mg/l, preferably at a concentration of 150 to 300 mg/l.

7. Culture medium according to any one of Claims 1 to 6, additionally containing a carbohydrate which can be metabolized by *Listeria ivanovii* but not by *Listeria monocytogenes.*

8. Culture medium according to Claim 7, in which the said carbohydrate which can be metabolized by *Listeria ivanovii* but not by *Listeria monocytogenes* is xylose.

9. Culture medium according to either of Claims 7 and 8, in which the concentration of the said carbohydrate is between 5 and 15 g/l.

10. Culture medium according to one of Claims 7 to 9, additionally containing a pH indicator, preferably Phenol Red.

11. Culture medium according to Claim 10, in which the concentration of the said pH indicator is between 50 and 300 mg/l.

12. Culture medium according to any one of Claims 1 to 11, additionally containing one or more antibacterial and/or antifungal agents, preferably chosen from amongst lithium chloride, acriflavine hydrochloride, nalidixic acid, polymixin B, cefotan, colistin sulphate, fosfomycin, ceftazidime, moxalactam, cycloheximide and amphotericin B.

13. Procedure for identification of the pathogenic bacteria of the genus *Listeria*, comprising:
- the inoculation of a sample liable to contain the said pathogenic bacteria of the genus *Listeria* onto an agar culture medium according to any one of Claims 1 to 12,
- the incubation of the said inoculated culture medium with the said sample,
- and the determination of the presence of the said pathogenic bacteria of the genus *Listeria* by the characteristic colour of the substrate.

14. Procedure according to Claim 13 in which the said sample liable to contain pathogenic bacteria is previously concentrated before being inoculated onto a culture medium according to any one of Claims 1 to 12.
